# EUROPEAN PATENT SPECIFICATION

(11) **EP 1 760 160 B1**
(45) Date of publication and mention of the grant of the patent: **24.03.2010**
(21) Application number: 06010796.8
(22) Date of filing: 26.05.2006
(51) Int. Cl.: C12Q 1/68

(54) **Methods and kits for detecting genetically modified organisms (GMO)**
Verfahren und Kits zum Nachweis genetisch veränderter Organismen (GVO)
Méthodes et compositions pour la détection d'organismes génétiquement modifiés (OGM)

(30) Priority: 08.06.2005 US 147299
(43) Date of publication of application: 07.03.2007
(73) Proprietor: AsiaGen Corporation, Hsin-Shi 74147 Tainan Hsien (TW)
(72) Inventor: Chou, George Chin-Sheng, Hsin-Shi 74147 Tainan Hsien (TW); Ko, Ni-Chin, Hsin-Shi 74147 Tainan Hsien (TW)
(74) Representative: Viering, Jentschura & Partner

(56) References cited:
- WO-A2-03/002762
- CN-A- 1 584 049
- US-A- 6 057 107
- US-A1- 2003 198 943
- WINDELS P ET AL: "Characterisation of the Roundup Ready soybean insert" EUROPEAN FOOD RESEARCH AND TECHNOLOGY, SPRINGER, HEIDELBERG, DE, vol. 213, no. 2, August 2001 (2001-08), pages 107-112, XP001145520 ISSN: 1431-4630
- DATABASE EMBL NUCLEOTIDE [Online] EMBL; 5 June 2003 (2003-06-05), WINDELS P, TAVERNIERS I, DEPICKER A, VAN BOCKSTAELE E, DE LOOSE M: "Synthetic construct for E35S promotor/plant junction region" XP002424130 retrieved from HTTP://WWW.NCBI.NLM.NIH.GOV/ENTREZ/QUERY.F CGI?CMD=SEARCH&DB=NUCLEOTIDE Database accession no. AJ308514
- DATABASE EMBL NUCLEOTIDE [Online] EMBL; 5 June 2004 (2004-06-05), SHAO BY, CHEN WB, JIANG SX, LI SS: "Glycine max transgenic cultivar Roundup Ready CP4-EPSPS precursor gene, partial cds." XP002424131 retrieved from HTTP://WWW.NCBI.NLM.NIH.GOV/ENTREZ/QUERY.F CGI?CMD=SEARCH&DB=NUCLEOTIDE Database accession no. AY596948
- VAN DUIJN G, HESSING M, VAN DER KAMP JW: "Identification de plantes transgeniques dans les aliments." COMPTES RENDUS DE L'ACADEMIE D'AGRICULTURE DE FRANCE, ACADEMIE D'AGRICULTURE DE FRANCE, PARIS, FR, vol. 83, no. 4, 1997, pages 153-157, XP001009667 ISSN: 0989-6988
- DATABASE EMBL NUCLEOTIDE [Online] EMBL; 26 April 2004 (2004-04-26), YING Z: "Glycine max transgenic strain Roundup Ready Agrobacterium tumefaciens NOS gene, 3' UTR." XP002424132 retrieved from HTTP://WWW.NCBI.NLM.NIH.GOV/ENTREZ/QUERY.F CGI?CMD=SEARCH&DB=NUCLEOTIDE Database accession no. AY578916
- DATABASE EMBL NUCLEOTIDE [Online] EMBL; 5 June 2003 (2003-06-05), WINDELS P, TAVERNIERS I, DEPICKER A, VAN BOCKSTAELE E, DE LOOSE M: "Synthetic construct for NOS 3'UTR/plant junction region." XP002424133 retrieved from HTTP://WWW.NCBI.NLM.NIH.GOV/ENTREZ/QUERY.F CGI?CMD=SEARCH&DB=NUCLEOTIDE Database accession no. AJ308515
- ERMOLLI M, FANTOZZI P: "An Innovative High-Throughput Assay for the Detection and the Quantification of GMOs Proteins and Sequences in Food Samples" 7 September 2005 (2005-09-07), IHCP-JRC, ISPRA (IT) - UNIVERSITY OF PERUGIA (IT), POSTER PRESENTATION , 10TH WORKSHOP ON THE DEVELOPMENTS IN THE ITALIAN PHD RESEARCH IN FOOD SCIENCE AND TECHNOLOGY, FOGGIA (IT) , XP002413537 Conference poster available online at http://biotech.jrc.it/home/docs.htm#poster s2005
- SMITH P L ET AL: "A rapid, sensitive, multiplexed assay for detection of viral nucleic acids using the FlowMetrix system" CLINICAL CHEMISTRY, AMERICAN ASSOCIATION FOR CLINICAL CHEMISTRY, WASHINGTON, DC, US, vol. 44, no. 9, 1998, pages 2054-2056, XP002170109 ISSN: 0009-9147
- WEDEMEYER N ET AL: "Flow cytometry: an 'old' tool for novel applications in medical genetics" CLINICAL GENETICS, COPENHAGEN, DK, vol. 60, July 2001 (2001-07), pages 1-8, XP002259894
- ENGEL KH, MOREANO F: "Methods to Detect the Application of Genetic Engineering in Composed and Processed Foods. In: HELLER KJ (Ed.): Genetically Engineered Food" 28 January 2005 (2005-01-28), WILEY-VCH , HEIDELBERG , XP002413538 * page 205 - page 230 * * paragraph [11.4.2] *

### SQUENCE LISTING

<110> Asiagen Corporation
   <120> Methods and kit for detecting genetically modified organism (GMO)
   <130> 2111-2
<140> EP 06010796
   <141> 2006-05-26
<150> US 11/147299
   <151> 2005-06-08
<160> 15
<170> PatentIn version 3.4
<210> 1
   <211> 20
   <212> DNA
   <213> Artificial
<220>
   <223> primer (35S F2)
<220>
   <221> misc_feature
   <222> (1)..(20)
<400> 1
   gaaaaggaag gtggctccta 20
<210> 2
   <211> 20
   <212> DNA
   <213> Artificial
<220>
   <223> primer (35S R2)
   <220>
   <221> misc_feature
   <222> (1)..(20)
<400> 2
   tccatctttg ggaccactgt 20
<210> 3
   <211> 20
   <212> DNA
   <213> Artificial
<220>
   <223> primer (CP4 F1)
<220>
   <221> misc_feature
   <222> (1)..(20)
<400> 3
   cccaagttcc taaatcttca 20
<210> 4
   <211> 20
   <212> DNA
   <213> Artificial
<220>
   <223> primer (CP4 R1)
<220>
   <221> misc_feature
   <222> (1)..(20)
<400> 4
   ccgtgaagca tgcaggctgt 20
<210> 5
   <211> 20
   <212> DNA
   <213> Artificial
<220>
   <223> primer (nos F1)
<220>
   <221> misc_feature
   <222> (1)..(20)
<400> 5
   gaatcctgtt gccggtcttg 20
<210> 6
   <211> 23
   <212> DNA
   <213> Artificial
<220>
   <223> primer (nos R2)
<220>
   <221> misc_feature
   <222> (1)..(23)
<400> 6
   gcgggactct aatcataaaa acc 23
<210> 7
   <211> 21
   <212> DNA
   <213> Artificial
<220>
   <223> primer (nosjun-B-F1) (EVENT SPECIFIC)
<220>
   <221> misc_feature
   <222> (1)..(21)
<400> 7
   tgaccctaat aggcaacagc a 21
<210> 8
   <211> 20
   <212> DNA
   <213> Artificial
<220>
   <223> primer (nosjun-B-R1) (event-specific)
<220>
   <221> misc_feature
   <222> (1)..(20)
<400> 8
   ccattcgcct caaacagttc 20
<210> 9
   <211> 20
   <212> DNA
   <213> Artificial
<220>
   <223> primer (lee F2)
<220>
   <221> misc_feature
   <222> (1)..(20)
<400> 9
   catctaaatg tgacagatcg 20
<210> 10
   <211> 21
   <212> DNA
   <213> Artificial
<220>
   <223> primer (lee MP2)
<220>
   <221> misc_feature
   <222> (1)..(21)
<400> 10
   gcgatcgagt agtgagagtc g 21
<210> 11
   <211> 19
   <212> DNA
   <213> Artificial
<220>
   <223> probe (35S P4)
<220>

   <221> misc_binding
   <222> (1)..(19)
<400> 11
   atcattgcga taaaggaaa 19
<210> 12
   <211> 20
   <212> DNA
   <213> Artificial
<220>
   <223> probe (CP4 P)
<220>
   <221> misc_binding
   <222> (1)..(20)
<400> 12
   gttttgttcc tttaggattt 20
<210> 13
   <211> 20
   <212> DNA
   <213> Artificial
<220>
   <223> probe (nos P2)
<220>
   <221> misc_binding
   <222> (1)..(20)
<400> 13
   catgacgtta tttatgagat 20
<210> 14
   <211> 20
   <212> DNA
   <213> Artificial
<220>
   <223> probe (nosjun P1) (event-specific)
<220>
   <221> misc_binding
   <222> (1)..(20)
<400> 14
   caaaactatt tgggatcgga 20
<210> 15
   <211> 20
   <212> DNA
   <213> Artificial
<220>
   <223> probe (lec P3)
<220>
   <221> misc_binding
   <222> (1)..(20)
<400> 15
   gaaggaagaa agtgtaataa 20

## Description

### Field of the invention

This invention relates to a method for detecting the presence and content of genetically modified soybean. This invention further relates to a kit for detecting the presence and content of genetically modified soybean.

### Description of Prior Art

Genetically modified organism (GMO) is a common term for a plant developed by a genetic engineering technology having genes or characteristics that are not generated by its original reproduction system, in order to enhance the convenience of distribution and process and to increase of output.

Characteristics of generally modified crops include increased output, resistance to damage by harmful insects, a high concentration of agricultural chemicals, deterioration during a long period of distribution, and improved shape, color and taste, and genetically modified organisms having more various characteristics can be developed in the future.

Commercialization of genetically modified organisms has rapidly progressed since Calgene developed tomatoes that are not easily crushed in 1994, and GMO soybeans of the Monsanto Company and corn of Novartis were fully commercialized in 1996. Although genetically modified organism seeds were initially much more expensive than natural seeds, the culture of genetically modified organisms rapidly increased with a lack of legislative regulations and expectations about its effect on people. Because farmers could save expenses on agricultural chemicals and fertilizers, and damage by harmful insects was reduced, commercially available genetically modified organisms inspected by the FDA from 1994 to 1998 numbered 39, including corns, tomatoes, potatoes, and soybeans, and another 30 are expected to be commercially available.

Because of saving on herbicide use and producing more output with less labor and expense, there are economic advantages to both companies and farmers in their production to solve the food shortage and environmental problems. However, the opposite opinion says that genetically modified organisms are not confirmed safe as food, and the culture of genetically modified organisms over a long period of time will cause a disturbance of the ecosystem and destruction of species diversity such that ultimately there will be a harmful influence on people, Given the above, introduction of labeling that indicates the presence of genetically modified organisms is also under dispute.

In European Union (EU), the GMOs are approbated formally to culture and sell in May 2004, however the foodstuff which contains more than 1% GMOs is requested strictly to label. Therefore, detection method for genetically modified DNA and protein should be systemized.

Methods employed for the confirmation of gene manipulation of a plant, include a method for detecting DNA and a method for detecting protein in genetically modified organisms. The common technologies for detecting GMO including (1) detection of specific nucleic acid, such as PCR, LCR (ligase chain reaction), NASBA (nucleic acid sequence-based amplification), and fingerprinting techniques; (2) detection of specific protein, such as 2D-SDS(two-dimensional sodium dodecyl sulfate polyacrylamide gel), western blot, and ELISA (enzyme linked immunosorbent assay); and (3) detection of specific enzyme activity. The PCR is used most widely in all detection technologies, based on its sensitivity, however, the disadvantages of PCR detection are sample contamination, the effects of PCR reagents and machines, and limitations of highly processed foods. The immunoassay has the advantages including low interference and less detection time, but low sensitivity and denatured proteins containing are the limitations.

The representative example of the DNA detection method is a genetically modified organism test kit of the Takara Company and it carries out PCR by way of a specific primer that amplifies foreign genes within a genetically modified organism. However, it takes a long time to test, has low reproducibility and cannot confirm whether foreign protein is actually expressed or not. The protein detection method uses an EPSPS (enolpyruvylshikimate-3-phosphate synthase) detection kit developed by SDI, but it cannot detect processed foodstuff. Although the protein detection method is relatively quick and has good reproducibility for seed in general, it is difficult to detect foreign protein in processed foodstuff prepared from a genetically modified organism.

It still requires a new technology to detect genetically modified organisms and foodstuff prepared from genetically modified organisms.

### Brief Description of the Drawings

**Figure 1** shows the DNA extraction results by using the Qiagen DNeasy^{®} Plant Mini Kit. M is marker; lane 1 is the DNA extracted from GM soybean; lane 2 is the DNA extracted from tofu (soybean curd), and lane 3 is the DNA extracted from soybean milk.

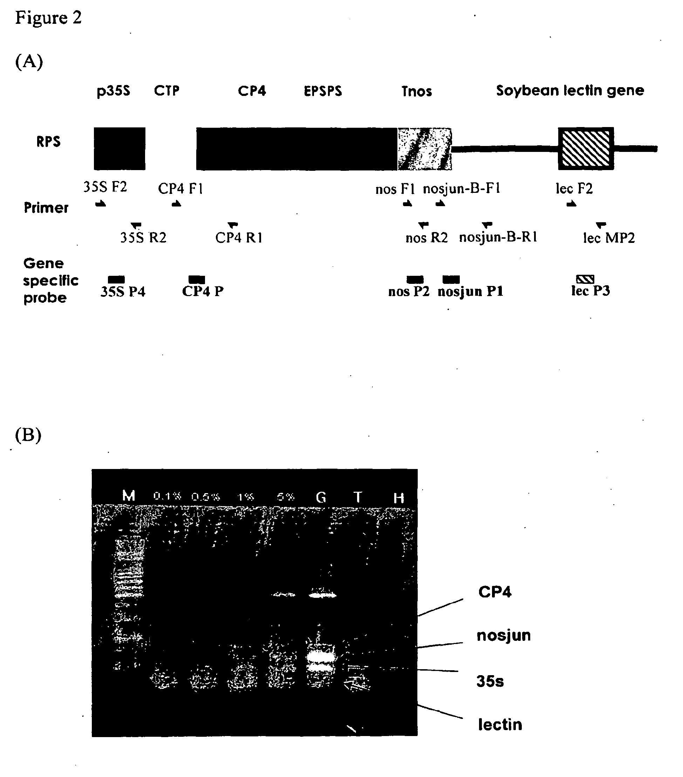

**Figure 2 (A)** illustrates the target of multiplex PCR and location of primers and probes. p35s is portion of CaMV35S promoter; CTP is chloroplast transit peptide; CP4 EPSPS is Agrobacterium sp. strain CP4 5-enolpyruvylshikimate-3- phosphate synthase; Tnos is portion of 3' non-translated region of nopaline synthase, and lectin is soybean specific gene SEQ ID NO 1: 35S F2; SEQ ID NO 2: 35S R2; SEQ ID NO 3: CP4 F1; SEQ ID NO 4: CP4 R1; SEQ ID NO 5: nos F2; SEQ ID NO 6: nos R2; SEQ ID NO 7: nosjun-B-F1; SEQ ID NO 8: nosjun-B-R1; SEQ ID NO 9: lec F2; SEQ ID NO 10: lec MP2; SEQ ID NO 11: 35S P4; SEQ ID NO 12: CP4 P; SEQ ID NO 13: nos P; SEQ ID NO 14: nosjun P; SEQ ID NO 15: lec P3) ; and **(B)** shows the multiplex PCR result, the arrowheads indicate the four amplified PCR products. H is H₂O; T is non-GM soybean; G is GM soybean; and the percentage 0.1 %, 0.5%, 1 % and 5 % means the GM soybean content in each sample.
**Figure 3** illustrates the results of GM soybean detection. CP4, nosjun, 35s and lectin are the probes used in the experiment respectively. The percentage 0.1 %, 0.5%, 1 %, 5 % and 100 % means the GM soybean (RRS) content in the sample.
**Figure 4** shows the probe specificity to the amplified PCR product. Multiplex is directed to four primer sets for PCR in one sample. Uni- is directed to single primer set for PCR and the primer is signed after the dash mark. GM is directed to genetically modified soybean as PCR sample. Non-GM is directed to non-genetically modified soybean as PCR sample.

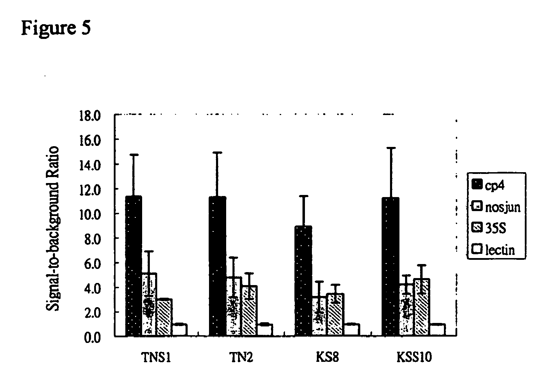

**Figure 5** shows the test results of various events of non-GM soybean using as background. TNS1, TN2, KS8, KSS10 are non-GM soybeans.
**Figure 6** shows the detection results of soybean and processed soybean products. TouFu is soybean curd. TouFu claimed as GM or Non-GM, fermented-TouFu and Misso are purchased from the commercial products. Fragrant-TouFu and Dehrdrated-TouFu are obtained from traditional market.
**Figure 7** shows standard curve of GM Soybean reference DNA. Filled circle: containing 0 % GM Soybean reference DNA, open square: containing 1 % GM Soybean reference DNA, filled triangle: containing 2 % GM Soybean reference DNA and open circle: containing 5 % GM Soybean reference DNA.

### Summary of the Invention

This invention provides a method for detecting genetically modified soybean as referred to in claims 1 to 8.

The invention further provides a kit for detecting GM soybean as referred to in claims 9 to 12 and the use of the kit as referred to in claims 13 to 15.

### Detailed Description of the Invention

This invention relates a method for detecting a genetically modified organisms (GMO), namely genetically modified soybean. The method is convenient for manipulation, and improves the disadvantages of traditional detection methods. This method improves the sensitivity and specificity of traditional polymerase chain reaction (PCR) by using multiplex target amplification and multiple probes confirmation. Accordingly, the present method could be applied for qualitative and quantitative analysis.

The method for detecting the GMO comprises (a) amplifying several transgenes of GMO by primer sets, (b) hybridizing the amplified products with probes, and (c) detecting the hybrids, wherein the primer set is labeled with biotin and the probe is labeled with colored bead. The amplifying step is using multiplex PCR to amplify multiple targets of DNA fragments in one sample, and the detection of hybrids is by the use of Luminex^{®} system to detect the colored bead. The characteristics of this method include the primer sets and the probes, which are labeled with special compounds for detection easily. The primer sets used in this invention are labeled with biotin, which is detectable by responding to avidin or avidin-like molecule. The probes used in this invention are labeled with colored beads, which are detectable by Luminex^{®} system. One probe is designed specifically to one PCR product, and different kinds of probes are labeled with different colored beads, respectively. Further, the detection method of this invention is a quantitative determination of the GMO contents in the sample.

The method of this invention can be used to detect the specific target gene(s), especially the transgene of soybean. The transgene is artificial insertion and not found in a non-genetically modified organism. The transgene is the foreign gene fragment and does not represent in the organism in natural evolution. The transgenes can be selected from anti-pest genes, anti-pathogen genes, anti-virus genes, anti-herbicide genes, stress-resistant genes, stress-tolerance genes, growth-regulation genes, and nutrient- enhancement genes. The mainly characteristic of transgene is not represented in a non-genetically modified organism (non-GMO). Based on the characteristic, the tansgene can be the detecting target to differentiate GMO and non-GMO. Selecting one or more target gene is allowed in this invention, and the more target genes, the more degrees of the specificity.

The polynucleotides used for primer or probe can be designed based on the target gene which is selected from the groups consisting of anti-pest gene, anti-pathogen gene, anti-virus gene, anti-herbicide gene, stress-resistant gene, stress-tolerance gene, growth-regulation gene, and nutrient- enhancement gene. The primer set is consisting of an upper primer and a lower primer, that flank a particular nucleotide sequence in the 5' and 3' position, respectively, and are used to amplify the target gene sequence. The primer set can be designed to amplify only the transgene fragment, or to amplify the gene fragment over the insertion site both including the transgene and the natural gene in the organism. The latter amplified gene fragment is transformation event specific, and it is a significant marker of genetic modification. In this invention, the primer is labeled with biotin, and the labeled biotin can be detected by adding the substrate avidin or avidin-like. The labeled primer used in amplification process such as polymerase chain reaction (PCR) can be detected for checking the amplification process is done or for double confirming with the probe hybridization. The sequence of probe is designed from the predicted amplified gene fragment sequence, and it is contained within the amplified target gene fragment. The probe is designed for hybridizing with the target gene specifically. In this invention, the probe is labeled with colored bead, and each one probe is labeled with unique color. The hybridization amount of probe and amplified fragment can be detected by detecting of the degree of color, so that the amplified target gene can be quantitative determined. In this invention, the GMO detection method detects not only the existence but also the content of GMO.

In the GMO detection, using the amplification and hybridization to detect the target tansgene of GMO further comprises selecting a natural gene for reference gene. The reference gene can be an indicator to confirm the accuracy of detection, and further can be a parameter to count the amount of the target gene(s). All genes existing in the species naturally can be selected to be the reference gene, and the natural gene near the target tansgene is the better choice. The primer set and probe are designed to amplify and hybridize the reference gene, respectively. The amplified reference gene can be a whole gene or a partial gene fragment, based on the size of target gene(s). In one embodiment, the reference gene is lectin.

The sample used in this invention is fresh crop, fresh food, or processed food. The sample also can be plant tissue or processed plant, and the most important term of the sample is to extract genomic DNA. The plant tissue can be from root, leaf, stem, flower, fruit or seed. The crop can be fresh, dried or processed. The processed food here means that the fresh plant (crop) or plant (crop) material is via artificial processing, such as air-dry, dehydration, refrigeration, or preservation. In the present invention the GMO is from soybean. It is also described herein that the GMO is from corn. Quantitative determination of the GMO contents in the sample is the target of this invention. First, extract the genomic DNA from the sample, and amplify the selected target gene fragment(s) by designed primer set(s). Then hybridize the amplified products with the designed probe(s), and detect the hybridization result to account the GMO content.

Also described herein is a polynucleotide for detecting a transgene of genetic modified soybean (GM soybean) comprising nucleotide sequence shown in SEQ ID Nos. 1, 2, 3, 4, 5, 6, 7, 8, 11, 12, 13, or 14. The polynucleotide comprising nucleotide sequence shown in SEQ ID Nos. 1, 2, 3, 4, 5, 6, 7, or 8 is labeled with biotin and is used for amplifying the transgene of GM soybean. Additionally, the polynucleotide comprising the nucleotide sequence shown in SEQ ID Nos. 11, 12, 13, or 14 is labeled with colored bead and is used for confirming the transgene of GM soybean. The primer and probe can detect the presence of the inserted gene, CP4 EPSPS (Agrobacterium sp. Strain CP4 5-enolpyruvylshikimate-3-phosphate synthase).

This invention further provides a kit for detecting GM soybean as referred to in claim 9. In one embodiment the kit comprises (a) biotin-labeled primer sets for amplifying transgene fragments of GM soybean, and (b) colored bead-labeled probes for confirming the amplified products; wherein the primer sets comprise the polypeptide sequence shown in SEQ ID Nos. 1∼8, and the probes comprise the polypeptide sequence shown in SEQ ID Nos. 11∼14. The primer sets and probes can detect the presence of the target transgene, CP4 EPSPS, in GM soybean. The kit further comprises a biotin-labeled primer set for amplifying a reference lectin gene of soybean, the primer set comprises the polypeptide sequence shown in SEQ ID Nos. 9 and 10. The kit further comprises a colored bead-labeled probe for confirming a reference lectin gene of soybean, the probe comprises the polypeptide sequence shown in SEQ ID No. 15.

The colored bead-labeled probe is detected by Luminex^{®} system, and the result can be used for accounting the GM soybean content. Based on the reference lectin gene and comparing the presence of transgene in samples, the kit can differentiate between GM soybean and non-GM soybean. Further, using the hybridization result of lectin gene as a parameter and calculating the hybridization result of transgene, the ratio of GM soybean can be obtained. Therefore, the kit is used for quantitatively determination of the GM soybean in the sample. The sensitivity of quantitatively detection of GM soybean content by using this kit can reach to 0.1 %.

The sample used in this kit is fresh soybean, soybean tissue, processed soybean, or processed food from soybean. The soybean tissue includes the tissue from root, leaf, stem, flower, fruit or seed of soybean. The soybean can be fresh, dried, or processed. The processed food from soybean can be processed via artificial processing, such as air-dry, dehydration, refrigeration, preservation, or even fermentation. The processed food from soybean, for example but not for limitation, includes soybean milk, tofu (soybean curd), fermented soybean curd, dried soybean curd, or soybean cake.

The following examples are offered by way of illustration and not by way of limitation.

### Example

### Example 1 Extraction of genomic DNA of soybean by using Qiagen DNeasy Plant Mini Kit

The tofu was cut into very small pieces. 100 mg tofu was added in a 1.5 ml microcentrifuge tube. All centrifugation steps are carried out at room temperature (15-25°C). 400 µl buffer AP1 were added into the tube, and the tube was vortexed vigorously. The tube was incubated at 65°C for 10 min and was vortexed occasionally during incubation. If possible, 4 µl RNase A solution (100 mg/ml) as added and mixed. Then, 130 µl Buffer AP2 was added and the tube was mixed. The tube was incubated on ice for 5 min. The tube was centrifuged at 14,000 rpm (18,000 x *g*) for 5 min. The lysate to a QIAshredder™ Spin Column sitting in a 2 ml collection tube was applied and centrifuged at maximum speed for 2 min. Transfer flow-throw to new tube, add 1.5 fold of AP3/E buffer and mix well. The mixer will apply to DNeasy^{®} Mini Column and centrifuged at 14,000 rpm (18,000 x g) for 1 min. Wash with 500 µl of AW buffer twice and elute DNA with H₂O.

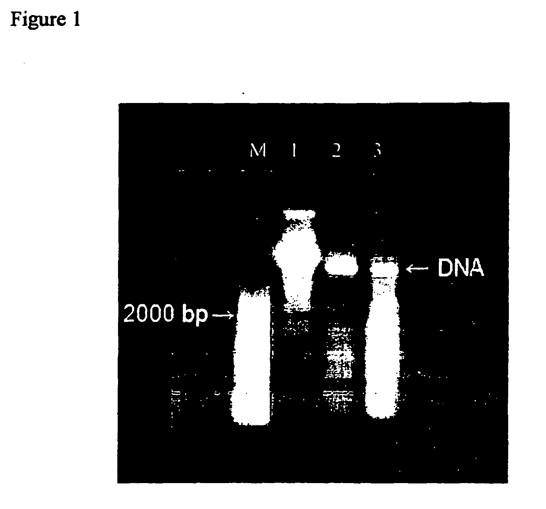

As showed in Figure 1, the DNA extraction results by using Qiagen DNeasy^{®}Plant Mini Kit were nice.

### Example 2 Amplification of PCR products

A new 0.2 ml tube was set up by adding up the reagents as follows:

| **Reagents** | **Brand Name** | **µl** | **final conc.** |
|---|---|---|---|
| 10x PCR buffer | PROMEGA | 5 µl | 1X |
| 25 mM MgCl₂ | PROMEGA | 4 µl | 2 mM |
| 10 mM dNTP Mix. | PROtech | 1 µl | 0.2 mM each |
| 10 µM 35sF2/R2 (SEQ ID Nos. 1 and 2) | Scino | 2 µl | 0.4 GM |
| 10 µM nosjun-B-F1/R1(SEQ ID Nos. 7 and 8) | Scino | 2 µl | 0.4 µM |
| 10 µM lecFd2/lecMP2 (SEQ ID Nos. 9 and 10) | Scino | 2 µl | 0.4 µM |
| 10 µM cp4F1/R1(SEQ ID Nos. 3 and 4) | Scino | 5 µl | 1.0 µM |
| 5 U/µl Tag | PROMEGA | 0.3 µl | 1.5 U |
| Genomic DNA | | | 100ng |

| | | | |
|---|---|---|---|
| Sterile water was added to final volume 50 µl. | | | |

After all reagents were added into the tube, the following protocol was performed.

| | **Temperature** | **Time** | **Cycle number** |
|---|---|---|---|
| 1 | 95°C | 5 min | 1 |
| 2 | 95°C | 30 sec | |
| | 55 °C | 45 sec | 35 |
| | 72 °C | 30 sec | |
| 4 | 72 °C | 5 min | 1 |

The test results were showed in Figure 2.

### Example 3 Hybridization and signal detection

The probes were 35sP4 (SEQ ID No. 11), cp4P (SEQ ID No. 12), nosjunP1 (SEQ ID No. 14) and lecP3 (SEQ ID No. 15), respectively. These probes were coupled with carboxylated microsphere ( MiraiBio Inc.) to form coupled probe.

33 µl Hybridization buffer, 1 µl probe 35sP4 coupled bead, 1µl probe cp4P coupled bead, 1 µl probenosjunP1 coupled bead and 1µl probe lecP3 coupled bead and 5 µl PCR product (0.1 %, 0.5%, 1 % and 5 % of GM soybean content in the sample) or control groups (GM soybean for positive control and non-GM soybean for negative control) were added into 1.5 ml tube, then mixed completely by vortex. The reaction mixture was kept at 46 °C for 15 min. Then, Spin down at 14000 rpm for 3 min, discard supernatant. Add 50 ul 1X TMAC, vortex, Spin down at 14000 rpm for 3 min. After spin, remove supernatant and add 50 ul of 4 ug/ml SA-PE in 1x TMAC, incubated at dark and room temperature for 10 min. Transfer 50 ul to 96-well plate and detection by Luminex^{®}.

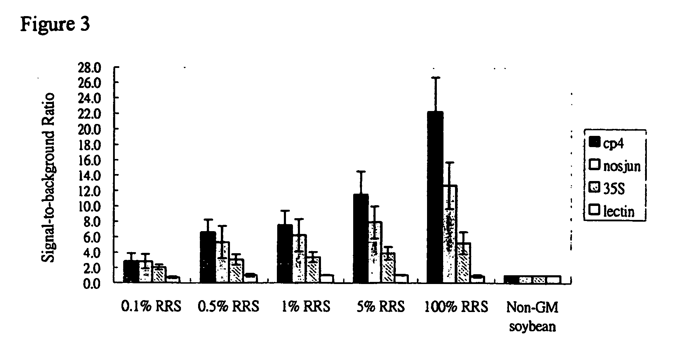

Following the above procedures, the non-GM soybean (KSS10), various GM soybean content (0.1 %, 0.5%, 1 %, 5% and 100 %) samples were assayed. The test results showed in Figure 3 that the method of the invention could simultaneously identify cp4, nosjun, 35s and lectin. Especailly, the method of the invention could determine the content of GM soybean as low as 0.1%.

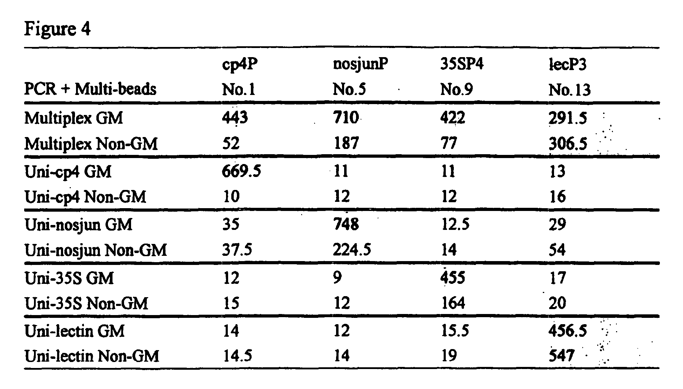

To further identify the probe specificity to the amplified PCR product, new test was directed to ten groups (Multiplex GM, Multiplex non-GM, Uni-cp4 GM, Uni-cp4 non-GM, Uni-nosjun GM, Uni-nosjun non-GM, Uni-35s GM, Uni-35s non-GM, Uni-lectin GM and Uni-lectin non-GM). Multi-beads are directed to four probe-coupled beads sets for luminex^{®} detection in one tube. Multiplex is directed to four primer sets for PCR in one sample. Uni- is directed to single primer set for PCR. GM is directed to genetically modified soybean as PCR sample. Non-GM is directed to non-genetically modified soybean as PCR sample. As showed in Figure 4, the probe specificity was very high in Uni-cp4 GM, Uni-nosjun GM, Uni-35s GM and Uni-lectin. As showed in Multiplex GM, it was surprised that non-cross reactions between in four specific probes were happened in the same tube.

### Example 4 Tests on different events of Non-GM soybean

Due to the non-GM soybean has been served as background, various events of non-GM soybean have been tested so as to further find out if they will affect the signal-to-background ratio. 4 different events of non-GM soybean have been selected in this test. As shown in this figure, all the results of signal-to-background ratio are rather similar by using those 4 events of non-GM soybean as background.

### Example 5 Tests on soybean and processed soybean products

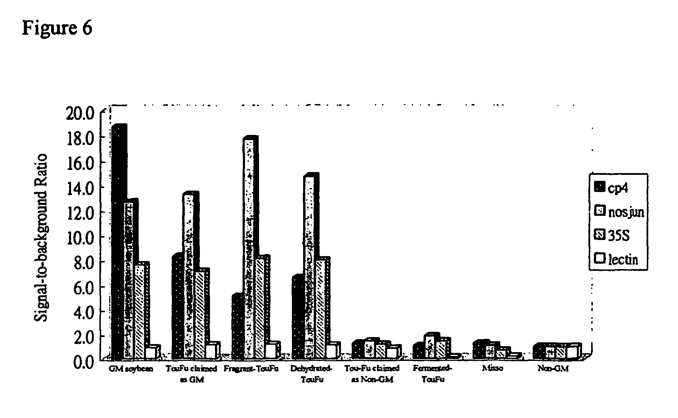

Various soybean products such as TouFu (soybean curd) fragrant-ToFu, dehydrated-TouFu, fermented-TouFu and Misso were purchased from market to assay whether the method of the invention could identify processed GM soybean. It clearly showed in Figure 6 that the processed GM soybean could be identified by the invention. However, highly processed soybean food such as Fermented-TouFu and Misso, could not be detected due to serious degradation of DNA after a long-term fermentation.

### Example 6 Standard curve of GM Soybean reference DNA

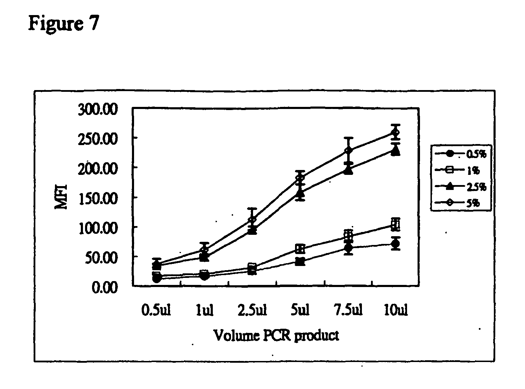

To explore the capability of this GMO monitoring system being able to provide quantitative analysis, a serial dilution of standard reference GM material were analyzed. The concentrations 0 %, 1 %, 2.5 % and 5 % of GM soybean DNA were used in experiment. Each concentration was tested in different volumes of DNA product, and the volumes were 0.5 µl, 1µl, 2.5µl, 5µl, 7.5µl and 10µl used in experiment. As shown in Figure 7, it clearly showed that the GM monitoring system could quantitatively analyze GM crop.

## Claims

1. A method for detecting genetically modified soybean comprising:
(a) amplifying transgenes of genetically modified soybean by using a primer set comprising SEQ ID NO: 1 and 2 for amplifying CaMV35S promoter, SEQ ID NO: 3 and 4 for amplifying CP4 EPSPS, and SEQ ID NO: 7 and 8 for amplifying Nopaline synthase terminator;
(b) hybridizing the amplified products with probes of SEQ ID NO: 11 (CaMV35S promotes), SEQ ID NO: 12 (CP4 EPSPS) and SEQ ID NO: 14 (Nopaline synthase terminator); and
(c) detecting the hybrids;
wherein the primer set of step (a) is labeled with biotin and the probes are labeled with colored bead.

2. The method as claimed in claim 1, further comprising the primer set of SEQ ID NO: 5 and 6 for amplifying Nopaline synthase and the probe of SEQ ID NO: 13 (Nopaline synthase).

3. The method as claimed in claim 1 or 2, which further comprises selecting a natural gene for reference gene.

4. The method as claimed in claim 3, wherein the reference gene is lectin.

5. The method as claimed in claim 1 or 2, wherein the step (a) is using multiplex PCR to amplify multiple targets of DNA fragments in one sample.

6. The method as claimed in claim 1 or 2, wherein the step (c) is using Luminex^{®} system to detect the colored bead.

7. The method as claimed in claim 1 or 2, wherein the detection is a quantitative determination of the GMO contents in the sample.

8. The method as claimed in claim 1 or 2, wherein the detected sample is fresh crop, fresh food, or processed food.

9. A kit for detecting GM soybean comprising:
(a) biotin-labeled primer sets for amplifying transgene fragments of GM soybean, and
(b) colored bead-labeled probes for confirming the amplified products; wherein the primer sets comprise the polynucleotide sequence shown in SEQ ID Nos. 1 to 4 and 7 to 8, and the probes comprise the polynucleotide sequence shown in SEQ ID Nos. 11 to 12 and 14.

10. The kit as claimed in claim 9, further comprising the primer set of SEQ ID NO: 5 and 6 for amplifying Nopaline synthase and the probe of SEQ ID NO: 13 (Nopaline synthase).

11. The kit as claimed in claim 9 or 10, which further comprises a biotin-labeled primer set for amplifying a reference lectin gene of soybean, the primer set comprises the polynucleotide sequence shown in SEQ ID Nos. 9 and 10.

12. The kit as claimed in claim 9 or 10, which further comprises a colored bead-labeled probe for confirming a reference lectin gene of soybean, the probe comprises the polynucleotide sequence shown in SEQ ID No. 15.

13. Use of a kit of claim 9 or 10, wherein the colored bead-labeled probes are detected by Luminex^{®} system.

14. Use of a kit of claim 9 or 10 for quantitative determination of the GM soybean content in the sample and for differentiating between GM soybean and non-GM soybean.

15. The use of claim 14, wherein the sample is fresh soybean, soybean tissue, processed soybean, or processed food from soybean.

## Patentansprüche

1. Ein Verfahren zum Detektieren einer genetisch veränderten Sojabohne, das umfasst:
(a) Amplifizieren von Transgenen einer genetisch veränderten Sojabohne durch Verwenden eines Primersatzes, der SEQ ID Nr: 1 und 2 zum Amplifizieren des CaMV35S Promotors, SEQ ID Nr: 3 und 4 zum Amplifizieren von CP4 EPSPS und SEQ ID Nr: 7 und 8 zum Amplifizieren des Nopalin Synthase Terminators umfasst;
(b) Hybridisieren der amplifizierten Produkte mit Sonden der SEQ ID Nr: 11 (CaMV35S Promotor), SEQ ID Nr: 12 (CP4 EPSPS) und SEQ ID Nr: 14 (Nopalin Synthase Terminator); und
(c) Detektieren der Hybride;
wobei der Primersatz aus Schritt (a) mit Biotin markiert ist und die Sonden mit gefärbten Kügelchen markiert sind.

2. Das Verfahren wie in Anspruch 1 beansprucht, des Weiteren umfassend den Primersatz der SEQ ID Nr: 5 und 6 zum Amplifizieren der Nopalin Synthase, und die Sonde der SEQ ID Nr: 13 (Nopalin Synthase).

3. Das Verfahren wie in Anspruch 1 oder 2 beansprucht, das weiterhin das Auswählen eines natürlichen Gens als Referenzgen umfasst.

4. Das Verfahren wie in Anspruch 3 beansprucht, wobei das Referenzgen Lectin ist.

5. Das Verfahren wie in Anspruch 1 oder 2 beansprucht, wobei der Schritt (a) Multiplex PCR verwendet, um mehrere Ziele von DNA Fragmenten in einer Probe zu amplifizieren.

6. Das Verfahren wie in Anspruch 1 oder 2 beansprucht, wobei der Schritt (c) das Luminex^{®} System zum Detektieren der gefärbten Kügelchen verwendet.

7. Das Verfahren wie in Anspruch 1 oder 2 beansprucht, wobei die Detektion eine quantitative Bestimmung des GVO Gehaltes in der Probe ist.

8. Das Verfahren wie in Anspruch 1 oder 2 beansprucht, wobei die detektierte Probe eine frische Pflanze, ein frisches Lebensmittel oder ein verarbeitetes Lebensmittel ist.

9. Ein Kit zum Detektieren einer GV Sojabohne, das umfasst:
(a) Biotin-markierte Primersätze zum Amplifizieren von transgenen Fragmenten einer GV Sojabohne, und
(b) gefärbte, mit Kügelchen markierte Sonden zum Bestätigen der amplifizierten Produkte; wobei die Primersätze die Polynukleotidsequenzen umfassen, die in SEQ ID Nr: 1 bis 4 und 7 bis 8 gezeigt werden, und die Sonden die Polynukleotidsequenzen umfassen, die in SEQ ID Nr: 11 bis 12 und 14 gezeigt werden.

10. Das Kit wie in Anspruch 9 beansprucht, des Weiteren umfassend den Primersatz der SEQ ID Nr: 5 und 6 zum Amplifizieren der Nopalin Synthase, und die Sonde der SEQ ID Nr: 13 (Nopalin Synthase).

11. Das Kit wie in Anspruch 9 oder 10 beansprucht, das weiterhin einen Biotin-markierten Primersatz zum Amplifizieren eines Referenz-Lectingens der Sojabohne umfasst, wobei der Primersatz die Polynukleotidsequenzen, wie in SEQ ID Nr: 9 und 10 gezeigt, umfasst.

12. Das Kit wie in Anspruch 9 oder 10 beansprucht, das weiterhin eine gefärbte, mit Kügelchen markierte Sonde zum Bestätigen eines Referenz-Lectingens der Sojabohne umfasst, wobei die Sonde die Polynukleotidsequenz, wie in SEQ ID Nr: 15 gezeigt, umfasst.

13. Verwendung eines Kits nach Anspruch 9 oder 10, wobei die gefärbten, mit Kügelchen markierten Sonden mittels des Luminex^{®}-Systems detektiert werden.

14. Verwendung eines Kits nach Anspruch 9 oder 10 zur quantitativen Bestimmung des GV Sojabohnengehaltes in der Probe und zum Differenzieren zwischen einer GV Sojabohne und einer nicht-GV Sojabohne.

15. Die Verwendung nach Anspruch 14, wobei die Probe eine frische Sojabohne, Sojabohnengewebe, eine verarbeitete Sojabohne oder ein verarbeitetes Lebensmittel aus Sojabohne ist.

## Revendications

1. Procédé de détection de soja génétiquement modifié comprenant :
(a) l'amplification de transgènes de soja génétiquement modifié en utilisant un ensemble d'amorces comprenant les SEQ ID NO: 1 et 2 pour amplifier le promoteur CaMV35S, les SEQ ID NO: 3 et 4 pour amplifier CP4 EPSPS, et les SEQ ID NO: 7 et 8 pour amplifier le terminateur de la Nopaline synthase ;
(b) l'hybridation des produits amplifiés avec des sondes de SEQ ID NO: 11 (promoteur CaMV35S), SEQ ID NO: 12 (CP4 EPSPS) et SEQ ID NO: 14 (terminateur de la Nopaline synthase) ; et
(c) la détection des hybrides ;
où l'ensemble d'amorces de l'étape (a) est marqué avec de la biotine et les sondes sont marquées avec une perle colorée.

2. Procédé selon la revendication 1, comprenant en outre l'ensemble d'amorces des SEQ ID NO: 5 et 6 pour amplifier la Nopaline synthase et la sonde de la SEQ ID NO: 13 (Nopaline synthase).

3. Procédé selon la revendication 1 ou 2, qui comprend en outre la sélection d'un gène naturel comme gène de référence.

4. Procédé selon la revendication 3, où le gène de référence est la lectine.

5. Procédé selon la revendication 1 ou 2, où l'étape (a) est l'utilisation de la PCR multiplex pour amplifier des cibles multiples de fragments d'ADN dans un seul échantillon.

6. Procédé selon la revendication 1 ou 2, où l'étape (c) est l'utilisation du système Luminex® pour détecter la perle colorée.

7. Procédé selon la revendication 1 ou 2, où la détection est une détermination quantitative des teneurs en organismes génétiquement modifiés dans l'échantillon.

8. Procédé selon la revendication 1 ou 2, où l'échantillon détecté est une récolte fraîche, un aliment frais, ou un aliment conditionné.

9. Kit de détection de soja génétiquement modifié comprenant :
(a) des ensembles d'amorces marquées à la biotine pour amplifier des fragments de transgène de soja génétiquement modifié, et
(b) des sondes marquées par des billes colorées pour confirmer les produits amplifiés ; où les ensembles d'amorces comprennent la séquence polynucléotidique représentée dans les SEQ ID NO: 1 à 4 et 7 à 8, et les sondes comprennent la séquence polynucléotidique représentée dans les SEQ ID NO: 11 à 12 et 14.

10. Kit selon la revendication 9, comprenant en outre l'ensemble d'amorces des SEQ ID NO: 5 et 6 pour amplifier la Nopaline synthase et la sonde de la SEQ ID NO: 13 (Nopaline synthase).

11. Kit selon la revendication 9 ou 10, qui comprend en outre un ensemble d'amorces marquées à la biotine pour amplifier un gène de lectine de référence du soja, l'ensemble d'amorces comprenant la séquence polynucléotidique représentée dans les SEQ ID NO: 9 et 10.

12. Kit selon la revendication 9 ou 10, qui comprend en outre une sonde marquée par des billes colorées pour confirmer un gène de lectine de référence du soja, la sonde comprenant la séquence polynucléotidique représentée dans la SEQ ID NO: 15.

13. Utilisation d'un kit selon la revendication 9 ou 10, où les sondes marquées par des billes colorées sont détectées par le système Lumineux®.

14. Utilisation d'un kit selon la revendication 9 ou 10 pour la détermination quantitative de la teneur en soja génétiquement modifié dans l'échantillon et pour faire la différence entre du soja génétiquement modifié et du soja non génétiquement modifié.

15. Utilisation selon la revendication 14, où l'échantillon est du soja frais, du tissu de soja, du soja conditionné, ou un aliment conditionné à base de soja.
